(19) Europäisches Patentamt
European Patent Office
Office européen des brevets

(11) Numéro de publication: **0 034 968**
**B1**

(12) **FASCICULE DE BREVET EUROPEEN**

(45) Date de publication du fascicule du brevet: **15.02.84**

(51) Int. Cl.[3]: **C 10 L 1/22**, C 08 G 65/32, C 07 D 207/416

(21) Numéro de dépôt: **81400215.0**

(22) Date de dépôt: **11.02.81**

(54) **Succinimides N-substitués, leur préparation et leur utilisation comme additifs pour carburants.**

(30) Priorité: **15.02.80 FR 8003459**

(43) Date de publication de la demande:
**02.09.81 Bulletin 81/35**

(45) Mention de la délivrance du brevet:
**15.02.84 Bulletin 84/7**

(84) Etats contractants désignés:
**AT BE DE GB IT NL SE**

(56) Documents cités:
**US - A - 3 897 454**
**US - A - 4 144 034**

(73) Titulaire: **INSTITUT FRANCAIS DU PETROLE**
**4, Avenue de Bois-Préau**
**F-92502 Rueil-Malmaison (FR)**

(73) Titulaire: **Sté ELF-FRANCE**
**137, rue de l'Université**
**F-75007 Paris (FR)**

(72) Inventeur: **Maldonado, Paul**
**20, rue Centrale**
**F-69360 St Symphorien D'Ozon (FR)**
Inventeur: **Cohen, Choua**
**111, Av. Barthélémy Buyer**
**F-69005 Lyon (FR)**
Inventeur: **Sillion, Bernard**
**93, rue Jolliot Curie**
**F-69005 Lyon (FR)**

**EP 0 034 968 B1**

Il est rappelé que: Dans un délai de neuf mois à compter de la date de publication de la mention de la délivrance du brevet européen toute personne peut faire opposition au brevet européen délivré, auprès de l'Office européen des brevets. L'opposition doit être formée par écrit et motivée. Elle n'est réputée formée qu'après paiement de la taxe d'opposition. (Art. 99(1) Convention sur le brevet européen).

Courier Press, Leamington Spa, England.

Succinimides n-substitués, leur préparation et leur utilisation comme additifs pour carburants

La présente invention concerne des succinimides N-substitués, leur préparation, leur utilisation comme additifs pour essence et les compositions d'essence améliorées résultantes, ayant de bonnes propriétés anti-rouille et de détergence dans le carburateur et dans les systèmes d'admission.

Un ralenti irrégulier et le calage des moteurs d'automobile à carburateur sont connus depuis longtemps comme des problèmes associés au fonctionnement de véhicules automobiles.

Une des causes du ralenti irrégulier et du calage est l'accumulation de dépôts sur le papillon du carburateur et sur la paroi environnante. L'accumulation de dépôts gêne l'écoulement normal de l'air dans le carburateur, conduisant à des mélanges riches en combustibles. Les dépôts peuvent être produits, par exemple, par l'accumulation d'impuretés ou de poussières provenant de l'air ou du recyclage des gaz de carter.

Par ailleurs, ces mélanges excessivement riches en combustible ont une combustion incomplète et de ce fait la pollution du l'air s'intensifie par l'accroissement de sa teneur en particules decarburant partiellement brûlé.

Les carburateurs modernes à grande capacité ont une structure complexe. Même s'il n'y a que peu de dépôt et de résidus, leur présence dans les organes de réglage fin de ces carburants pertube fortement le fonctionnement de ces derniers. Il en résulte en particulier une mauvaise composition du mélange carburant/air de telle sorte que le rapport $CO/CO_2$ augmente.

Les mesures en vue d'y remédier comprennent soit le nettoyage coûteux périodique du carburateur et des tulipes de soupapes d'admission, soit l'accélération du régime normal de ralenti, ce qui entraîne une plus grande difficulté dans la conduite du véhicule et une augmentation inutile de consommation en combustible.

Il est connu que les dépôts présents dans le carburateur peuvent être réduits ou que l'accumulation de ces dépôts peut être empêchée par l'utilisation de carburants qui contiennent certains additifs qu'on appelle détergents pour carburateurs.

En plus des additifs de détergence pour carburateurs, les carburants modernes exigent d'autres additifs pour améliorer le comportement du carburant, par exemple ceux qui fournissent une protection anti-rouille et une limitation de dépôts dans le dispositif d'admission. De préférence, les additifs doivent être des additifs multifonctionnels.

Bien que de nombreux additifs multifonctionnels soient suggérés dans la technique, beaucoup d'entre eux ne sont pas acceptables soit parce qu' ils entraînent des effets indésirables, soit parce qu'ils doivent être utilisés en quantité excessive pour fournir les propriétés désirées.

La présente invention a pour but de fournir une famille d'additifs présentant des caractéristiques multifonctionnelles, incluant des propriétés anti-rouille et de détergence dans le carburateur et en ne donnant pas de dépôts gênants sur les soupapes, utilisables à une concentration habituellement comprise entre 0,001 et 0,05% en poids par rapport au poids de mélange d'hydrocarbures sans que ces limites soient impératives.

D'une manière générale, ou peut définir les produits de l'invention comme étant obtenus par réaction d'anhydride maléique avec au moins un monoalcool oxyalcoylé ou poly-oxyalcoylé répondant à la formule générale:

$$R^1\text{-}(O\text{—}A)_n\ OH \qquad (I)$$

dans laquelle $R^1$ représente un radical aliphatique, linéaire ou ramifié, saturé ou insaturé, renfermant de 12 à 25, de préférence de 12 à 22 atomes de carbone; A représente un radical alkylène, linéaire ou ramifié, renfermant de 2 à 4 atomes de carbone, dont au moins deux sont en chaîne droite; et n est un nombre entier de 1 à 50;

le produit obtenu étant mis à réagir avec une amine monoprimaire répondant à la formule générale:

$$R^2\text{-}(X\text{—}B)_m\ NH_2 \qquad (II)$$

dans laquelle $R^2$ représente un radical aliphatique, linéaire ou ramifié, saturé ou insaturé, renfermant de 8 à 25, de préférence de 8 à 22 atomes de carbone; B représente un radical alkylène, linéaire ou ramifié, renfermant de 2 à 4 atomes de carbone, dont au moins deux sont en chaîne droite; X représente un groupe —NH— ou un atome d'oxygène —O—; et m est un nombre entier de 0 à 4.

Ces composés se distinguent des succinimides N-substitués décrits dans USA 3 897 454 pouvant être préparés par réaction de l'anhydride maléique sur un polyalkylèneglycol puis réaction du produit éthérifié obtenu avec une polyalkylènepolyamine.

Le produit selon la présente invention résultant de la réaction de l'anhydride maléique et d'un monoalcool oxyalcoylé ou polyoxyalcoylé de formule (I) peut être considéré comme répondant lui-même à la formule générale

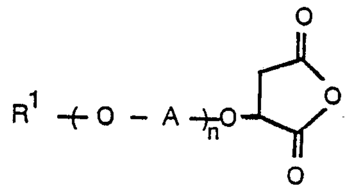

(III)

avec les mêmes notations que précédemment.

Dans le cas ou l'on met en jeu une amine monoprimaire de formule (II), en une proportion d'environ 1 mole par mole de l'anhydride représenté par la formule (III), le produit final pourra être considéré comme répondant à une formule du type:

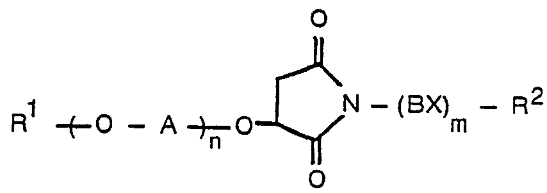

(IV)

acec les mêmes notations que précédemment.

Dans les monoalcools oxyalcoylés ou polyoxyalcoylés de formule (I) utilisés pour préparer les anhydrides représentés par la formule (III) le radical $R^1$ est de préférence un radical alkyle linéaire, renfermant 12 ou 13 atomes de carbone. En outre, ces produits portent avantageusement leur groupement hydroxylé terminal sur un atome de carbone secondaire ou tertiaire d'un groupement alcoylène: les chaînes de motifs alcoylène se terminent avantageusement par un motif oxyde de propylène

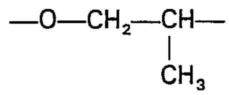

ou par un motif oxyde d'isobutylène

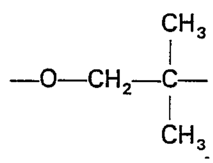

Parmi les monoalcools oxyalcoylés ou polyoxyalcoylés de formule (I) considérés dans l'invention, on peut citer comme exemples particulièrement avantageux:

l'alcool laurique polyoxypropylé, renfermant par exemple 21 motifs oxyde de propylène;
l'alcool tridécylique trioxypropylé;
l'alcool tridécylique à séquences oxyde d'éthylène et oxyde de propylène;
l'alcool tridécylique à séquences oxyde d'éthylène et oxyde d'isobutylène.

Parmi les amines de formule (II) utilisables pour préparer les produits de type (IV) de l'invention, on peut citer comme exemples particulièrement avantageux:

la N-oléyl propanediamine, de formule $C_{18}H_{35}NH—(CH_2)_3—NH_2$;
la N-isotridécyl propanediamine, de formule $C_{13}H_{27}NH—(CH_2)_3—NH_2$;
l'isotridécyloxy-3 propylamine-1, de formule $C_{13}H_{27}—O—(CH_2)_3—NH_2$;
l'undécyloxy-3 propylamine-1, de formule $C_{11}H_{23}—O—(CH_2)_3—NH_2$;
la tridécyloxy-3 propylamine-1, de formule $C_{13}H_{27}—O—(CH_2)_3—NH_2$; et
l'(éthyl-2 hexyl) oxy-3 propylamine-1, de formule

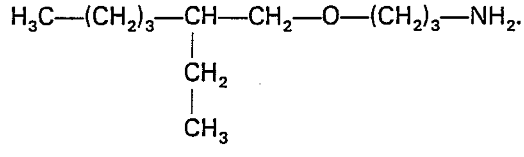

Pour préparer les produits de l'invention, on peut opérer la condensation des réactifs sans solvant, mais de préférence avec un solvant, par exemple un hydrocarbure aromatique de point d'ébullition compris entre 70°C et 200°C, en éliminant l'eau formée au cours de la réaction.

La température de réaction se situe habituellement entre 65°C et 200°C, et de préférence entre 80°C et 160°C. La durée de réaction est comprise entre 0,5 et 6 h et de préférence entre 1 h et 3 h. et 3 h.

Les amines de formule (II) sont habituellement utilisées à raison de 1,02 à 1,2 mole de préférence de 1,05 à 1,1 mole de l'anhydride représenté par la formule (III).

Pour obtenir les produits de l'invention, il est possible également de condenser en une seule étape l'anhydride maléique, le monoalcool oxyalcoylé ou polyoxyalcoylé de formule (I) et l'amine de formule (II). La structure "éthers-succinimides N-substitués" des produits de l'invention peut être confirmée par spectrométrie infra-rouge: les spectres infra-rouge présentent en effet des bandes d'absorption succinimides à 1700 $cm^{-1}$ et des bandes d'absorption éthers à 1110 $cm^{-1}$.

Ces produits sont utilisés comme additifs pour carburants. Ils présentent l'avantage de posséder des propriétés multifonctionnelles; ils exercent un effet tensio-actif, ils présentent de bonnes propriétés filmogènes et confèrent aux surfaces métalliques une résistance à la corrosion améliorée; ils présentent en outre une stabilité thermique suffisante pour ne pas contribuer par eux-mêmes à la formation de dépôts et ils empêchent, grâce à leur effet filmogène maintenu à haute température, les dépôts habituellement formés par les particules d'huile de graissage ou les produits aromatiques ou oléfiniques plus ou moins décomposés à chaud.

Les produits de l'invention, peuvent être utilisés dans les carburants automobiles à des concentrations par exemple de 10 à 500 et de préférence de 20 à 200 parties par million (ppm) en poids, sans formation de trouble, même à basse température, et ils peuvent être associés sans inconvénients aux autres additifs usuels.

Les exemples suivants illustrent l'invention, mais ils ne doivent en aucune manière être considérés comme limitatifs.

Exemple 1

Dans le réacteur de deux litres, on introduit 715 g (0,5 mole) de monolauryl- éther du polypropylène glycol (c'est-à-dire l'alcool laurique poly-oxypropylé contenant environ 21 motifs oxyde de propylène par molécule), puis on chauffe avec agitation sous azote à 185°C. On additionne alors 53,9 g (0,55 mole) d'anhydride maléique et on laisse réagir pendant 15 h à 185°C sous azote.

Après refroidissement, on récupère une huile jaune-orange, dont le spectre I.R. correspond à la structure d'un anhydride succinique polypropoxylé.

153,8 g de cette huile sont ajoutés à une solution de 39,6 g (0,12 mole) de N-oléyl propanediamine dans 250 ml de xylène.

Le mélange est chauffé à reflux pendant 3h 30 à 144°C, avec distillation azéotropique de l'eau formée au cours de la réaction. On obtient 378 g d'une solution à 50% dans le xylène d'un produit dont la structure de N-alkénylsuccinimide polypropoxylée est confirmée par spectrométrie I.R.

Exemple II

On dissout dans un réacteur de 500 ml, 9,8 g (0,1 mole) d'anhydride maléique et 145 g (0,1 mole) de mono-lauryl-éther de polypropylène glycol (c'est-à-dire d'alcool laurique polyoxypropylé contenant environ 21 motifs oxyde de propylène par molécule) dans 150 ml de xylène. Après un chauffage de 3h à reflux, le mélange est refroidi à température ordinaire, puis additionné de 36g (0,1 mole) de N-oléyl propanediamine.

Le mélange est chauffé à nouveau pendant 3h à reflux à 145°C, avec extraction azéotropique de l'eau formée.

On obtient une solution à 50% dans le xylène d'un produit dont la structure, mise en évidence par spectrometrie I.R., est identique à celle obtenue dans l'exemple précédent.

Tests sur les produits

Les produits préparés comme décrit dans les exemples I et II ont été utilisés comme additifs dans des essences, et l'on a déterminé les performances des essences ainsi additivées, dans un certain nombre de tests qui seront décrits ci-après:

a) Stabilité thermique par la procédure ISD

La procédure ISD (Induction System Deposit) est réalisée selon la méthode de laboratoire mise au point au Southwest Research Institute (San Antonio Texas) par A. A. JOHNSTON et E. DIMITROFF, SAE Transactions, Vol. 75, p. 885—891, Article 660 783 (1969).

Elle permet d'évaluer la stabilité thermique d'un additif en solution dans un supercarburant en simulant son passage sur les surfaces chaudes d'un moteur en fontionnement et en particulier sur les soupapes d'admission.

Les produits des exemples I et II ont été ajoutés à la concentration de 0,01% en poids dans le supercarburant. Les résultats sont indiqués au Tableau I ci-après. On donne aussi les résultats relatifs au supercarburant non additivé et les résultats relatifs à un supercarburant contenant la même concentration d'un additif commercial.

TABLEAU I

| Tests / Produits testés | Dépôts sur surfaces chaudes à 200°C en mg (tests ISD) |
|---|---|
| Supercarburant non additivé | 0 |
| Supercarburant + composé de l'exemple I à 0,01% | 0 |
| Supercarburant + composé de l'exemple II à 0,01% | 0 |
| Supercarburant + additif commercial A à 0,01% | 1,8 |

b) Test de corrosion et mesure de la tension interfaciale

Les produits des Exemples I et II ont encore été utilisés dans un super carburant à la concentration de 0,01% en poids.

Le test de corrosion consiste à étudier la corrosion, par de l'eau de mer synthétique, d'éprouvettes cylindriques en acier ordinaire poli, selon la norme ASTM D 665 modifiée (température 32,2°C; durée 20h).

La tension interfaciale a été mesurée selon la méthode ASTM D 971. Les résultats sont donnés au Tableau II, où on a également indiqué, à titre de comparaison les résultats obtenus avec le supercarburant non additivé et ceux obtenus avec le supercarburant contenant un additif commercial, à la concentration de 0,01% en poids.

TABLEAU II

| Tests / Produits testés | Corrosion % | Tension interfaciale eau-supercarburant en dynes/cm |
|---|---|---|
| Supercarburant non additivé | 100 | 38,2 |
| Supercarburant + composé de l'exemple I à 0,01% | 0,5 | 8,2 |
| Supercarburant + composé de l'exemple II à 0,01% | 0 | 9,4 |
| Supercarburant + additif commercial B à 0,01% | 95 | 31,5 |

c) Essais au banc d'encrassement du carburateur

L'essai au banc d'encrassement des carburateurs est réalisé selon la procédure BNPé R5 GTL mise au point par ELF/IFP.

La méthode consiste à apprécier sur un moteur au banc d'essai l'aptitude d'un carburant à maintenir en carburateur propre.

Le test dure 12 heures et comprend 2 périodes de 6 heures séparées par un arrêt de 18 heures. L'encrassement du carburateur est favorisé par recyclage à l'admission d'une fraction des gaz d'échappement.

Une technique de cotation visuelle du corps de carburateur exprime les résultats de façon quantitative: de 0 à 10.

10 représentant un carburateur neuf

0 un carburateur encrassé.

La cotation tient compte de l'existence, de la couleur et de la position des dépôts dans le carburateur et sur le volet d'admission.

Les produits des Exemples I et II ont été utilisés à raison de 0,0167% en poids par rapport au supercarburant. Les résultats sont donnés au tableau III, où on a également fait figurer, à titre de comparaison, les résultats obtenus avec le supercarburant non additivé et le supercarburant contenant un additif commercial, à la concentration de 0,0276% en poids.

TABLEAU III

| Essais moteurs / Produits testés | Encrassement carburateur Mérite final sur 10 |
|---|---|
| Supercarburant non additivé | 3,0 |
| Supercarburant + composé de l'exemple I à 0,0167% | 8,2 |
| Supercarburant + composé de l'exemple II à 0,0167% | 8,6 |
| Supercarburant + additif commercial B à 0,0276% | 7,45 |

d) Essais au banc d'encrassement des soupapes d'admission

L'essai au banc d'encrassement des soupapes d'admission est réalisé selon la méthode mise au point par le Département Recherche et Développement de la Deutsche BP Aktiengesellschaft à Hambourg.

Il a pour but de déterminer, au banc d'essai, l'aptitude des supercarburants additivés au maintien de la propreté des soupapes d'admission.

L'essai consiste à équiper un moteur 1.25 Opel Kadett d'un double carburateur. Ceci permet de tester simultanément soit un additif à deux concentrations différentes, soit deux additifs différents, soit un supercarburant additivé par rapport au même supercarburant non additivé.

L'essai simule une séquence de conduite à régime normal et au ralenti à 35,50 et 80 km à l'heure.

Le programme de fonctionnement au banc d'essai est le suivant: Durée de l'essai 40 heures:
30 secondes de relenti à 1000 tours par minute
1 minute à 3000 tours par minute (égal à 80 km à l'heure)
1 minute à 1300 tours par minute (égal à 35 km à l'heure)
1 minute à 1850 tours par minute (égal à 50 km à l'heure).

En fin d'essai, l'état des tulipes de soupapes d'admission est côté en milligrammes de dépôt par soupape, qui qualifiera le carburant contenant l'additif par rapport au carburant seul.

Les produits préparés dans les exemples I et II ont été utilisés à raison de 0,0167% en poids dans le supercarburant. Les résultats sont donnés au Tableau IV ci-après, où l'on a également fait figurer les résultats obtenus avec le supercarburant non additivé et ceux obtenus avec le supercarburant contenant, à la même concentration de 0,0167% en poids un additif commercial.

TABLEAU IV

| Essais moteurs / Produits testés | Encrassement soupapes d'admission en mg de dépôt/soupape |
|---|---|
| Supercarburant non additivé | 233 |
| Supercarburant + composé de l'exemple I à 0,0167% | 162 |
| Supercarburant + composé de l'example II à 0,0167% | 175 |
| Supercarburant + additif commercial A à 0,0167% | 259 |

## Revendications

1. Succinimide N-substitué caractérisé en ce qu'il résulte de la réaction de l'anhydride maléique avec au moins un monoalcool oxyalcoylé ou polyoxyalcoylé répondant à la formule générale:

$$R^1\!\left(O-A\right)_n OH$$

dans laquelle $R^1$ représente un radical aliphatique, linéaire ou ramifié, saturé ou insaturé, de 12 à 25 atomes de carbone; A représente un radical alkylène, linéaire ou ramifié, de 2 à 4 atomes de carbone, dont au moins 2 sont en chaîne droite; et n est un nombre entier de 1 à 50;
le produit étant mis à réagir avec une amine monoprimaire répondant à la formule générale:

$$R^2\text{-}(X\text{—}B)_m\ NH_2$$

dans laquelle $R^2$ représente un radical aliphatique, linéaire ou ramifié, saturé ou insaturé, renfermant de 8 à 25 atomes de carbone; B représente un radical alkylène, linéaire ou ramifié, renfermant de 2 à 4 atomes de carbone, dont au moins deux sont en chaîne droite; X représente un groupe —NH— ou un atome d'oxygène —O—; et m est un nombre entier de 0 à 4.

2. Succinimide N-substitué selon la revendication 1, caractérisé en ce que, dans ledit monoalcool oxyalcoylé ou polyoxyalcoylé, le radical $R^1$ est un radical alkyle linéaire renfermant 12 ou 13 atomes de carbone.

3. Succinimide N-substitué selon la revendication 2, caractérisé en ce que ledit monoalcool oxyalcoylé ou polyoxyalcoylé est choisi parmi l'alcool laurique polyoxypropylé, l'alcool tridécylique trioxypropylé, l'alcool tridécylique à séquences oxyde d'éthylène et oxyde de propylène et l'alcool tridécylique à séquences oxyde d'éthylène et oxyde d'isobutylène.

4. Succinimide N-substitué selon l'une des revendications 1 à 3, caractérisé en ce que ladite amine monoprimaire est choisie parmi la N-oléyl propanediamine, la N-isotridécyl propanediamine, l'isotridécyloxy-3 propylamine-1, l'undécyloxy-3 propylamine-1, le tridécyloxy-3 propylamine-1 et l'(éthyl-2 hexyl) oxy-3 propylamine-1.

5. Procédé de préparation d'un succinimide N-substitué selon l'une des revendications 1 à 4, caractérisé en ce que, dans une première étape, on fait réagir l'anhydride maléique avec au moins un monoalcool oxyalcoylé ou polyoxyalcoylé répondant à la formule générale:

$$R^1\text{-}(O\text{—}A)_n\ OH$$

et en ce que, dans une seconde étape, on fait réagir le produit résultant de la première étape avec une amine monoprimaire répondant à la formule générale $R^2\text{-}(XB)_m\ NH_2$, où $R^1$, A, n, $R^2$, X, B, et m sont définis comme dans la revendication 1.

6. Procédé selon la revendication 5, caractérisé en ce que l'on utilise dans la deuxième étape amine monoprimaire répondant à la formule générale $R^2\text{-}(X\text{—}B)_m\ NH_2$ en une proportion de 1,02 à 1,2 mole par mole du produit résultant de la première étape.

7. Procédé selon l'une des revendications 5 et 6, caractérisé en ce que l'on opère à une température de 65 à 200°C, en éliminant l'eau formée au cours des réactions.

8. Procédé selon l'une des revendications 5 à 7, caractérisé en ce que l'on opère en une seule étape.

9. Composition de carburant, caractérisée en ce qu'elle comprend une proportion majeure d'un carburant et une proportion de 10 à 500 ppm en poids d'un succinimide N-substitué selon l'une des revendications 1 à 4.

10. Composition de carburant selon la revendication 9, caractérisée en ce que la proportion de succinimide N-substitué est de 20 à 200 ppm en poids.

## Claims

1. N-substituted succinimide characterized in that it results from reacting maleic anhydride with at least one oxyalkylated or polyoxyalkylated monoalcohol of the general formula

$$R^1\text{-}(O\text{—}A)_n\ OH$$

wherein $R^1$ is a linear or branched, saturated or unsaturated aliphatic radical containing from 12 to 25 carbon atoms; A is a linear or branched alkylene radical having from 2 to 4 carbon atoms, at least two of which are in a straight chain and n is an integer from 1 to 50; the resulting product being reacted with a monoprimary amine of the general formula

$$R^2\text{-}(X\text{—}B)_m\ NH_2$$

wherein $R^2$ is a linear or branched, saturated or unsaturated, aliphatic radical containing from 8 to 25 carbon atoms; B is a linear or branched alkylene radical containing from 2 to 4 carbon atoms at least two of which are in a straight chain; X is a —NH— group or an oxygen atom —O—, and m is an integer from 0 to 4.

2. N-substituted succinimide according to claim 1, characterized in that in, said oxyalkylated or polyoxyalkylated monoalcohol, the radical $R^1$ is a linear alkyl radical comprising 12 or 13 carbon atoms.

3. N-substituted succinimide according to claim 2, characterized in that said oxyalkylated or polyoxyalkylated monoalcohol selected from polyoxypropylated lauric alcohol, trioxypropylated tridecyl alcohol, tridecyl alcohol with sequential recurrent units of ethylene oxide and propylene oxide and tridecyl alcohol with sequential recurrent units of ethylene oxide and isobutylene oxide.

4. N-substituted succinimide according to anyone of claims 1 to 3, characterized in that said monoprimary amine is selected from N-oleyl propanediamine, N-isotridecyl propanediamine, 3-isotridecyloxy 1-propylamine, 3-hendecyloxy 1-propylamine, 3-tridecyloxy 1-propylamine and 3-(2-ethyl hexyl)oxy 1-propylamine.

5. A process for the preparation of a N-substituted succinimide according to anyone of claims 1 to 4, characterized in that, in a first step, maleic anhydride is reacted with at least one oxyalkylated or polyoxyalkylated monoalcohol of the general formula:

$$R^1\text{-}(O\text{—}A)_n\ OH$$

and, in a second step, the product obtained in the first step is reacted with a monoprimary amine of the general formula

$$R^2\text{-}(X\text{—}B)_m\ NH_2$$

wherein $R^1$, A, n, $R^2$, X, B and m are defined as in claim 1.

6. A process according to claim 5, characterized in that, in the second step, the monoprimary amine of the general formula

$$R^2\text{-}(X\text{—}B)_m\ NH_2$$

is used in a proportion of from 1.02 mole to 1.2 mole per mole of the product obtained in the first step.

7. A process according to anyone of claims 5 and 6, characterized in that it is conducted at a temperature from 65 to 200°C and comprises the removal of the water formed during the reactions.

8. A process according to anyone of claims 5 to 7, characterized in that it is conducted in a single step.

9. Motor fuel composition characterized in that it comprises a major proportion of a motor fuel and from 10 to 500 ppm by weight of a N-substituted succinimide according to anyone of claims 1 to 4.

10. Motor fuel composition according to claim 9, characterized in that the proportion of N-substituted succinimide is from 20 to 200 ppm by weight.

**Patentansprüche**

1. N-substituiertes Succimid, dadurch gekennzeichnet, daß es aus der Reaktion von Maleinsäureanhydrid mit wenigstens einem oxyalkoylierte oder poly-oxyalkoylierten Mono-Alkohol entsprechend der allgemeinen Formel

$$R^1\text{-}(O\text{—}A)_n\ OH$$

erhalten wird, in der $R^1$ einen linearen oder verzweigten aliphatischen gesättigten oder ungesättigten Rest mit 12 bis 25 Kohlenstoffatomen darstellt, A einen linearen oder verzweigten Alkylenrest mit 2 bis 4 Kohlenstoffatomen, von denen wenigstens 2 in einer geraden Kette sind, und n eine ganze Zahl von 1 bis 50 ist, wobei das Produkt zur Reaktion gebracht wird mit einem monoprimären Amin entsprechend der allgemeinen Formel

$$R^2\text{-}(X\text{—}B)_m\ NH_2,$$

in der $R^2$ einen linearen oder verzweigten gesättigten oder ungesättigten aliphatischen Rest, umfassend 8 bis 25 Kohlenstoffatome, darstellt, B einen linearen oder verzweigten Alkylenrest mit 2 bis 4 Kohlenstoffatomen, von denen wenigstens zwei in einer geraden Kette sind, darstellt, X eine —NH-Gruppe oder ein Sauerstoffatom —O— darstellt, und m eine ganze Zahl von 0 bis 4 ist.

2. N-substituiertes Succinimid gemäß Anspruch 1, dadurch gekennzeichnet, daß in dem genannten oxyalkoylierten oder polyoxyalkoylierten Mono-Alkohol, der Rest $R^1$ ein linearer Alkylrest mit 12 oder 13 Kohlenstoffatomen ist.

3. N-substituiertes Succinimid gemäß Anspruch 2, dadurch gekennzeichnet, daß der genannte oxyalkoylierte oder polyoxyalkoylierte Monoalkohol gewählt ist unter polyoxypropyliertem Laurylalkohol, trioxypropyliertem Tridecylalkohol, Tridecylalkohol mit Ethylenoxid- und Propylenoxid-Sequenzen und Tridecylalkohol mit Ethylenoxid- und Isobutylenoxid- Sequenzen.

4. N-substituiertes Succinimid gemäß einem der Ansprüche 1 bis 3, dadurch gekennzeichnet, daß das genannte monoprimäre Amin gewählt ist unter dem N-Oleyl-Propandiamin, dem N-Isotridecyl-Propandiamin, dem Isotridecyloxy-3-Propylamin-1, dem Undecyloxy-3-Propylamin-1, dem Tridecyloxy-3-Propylamin-1 und dem (Ethyl-2-Hexyl)-Oxy-3-Propylamin-1.

5. Verfahren zur Herstellung eines N-substituierten Succinimids gemäß einem der Ansprüche 1 bis 4, dadurch gekennzeichnet, daß man in einer ersten Stufe das Maleinsäureanhydrid mit wenigstens einem oxyalkoylierten oder propyl-oxyalkoylierten Mono-Alkohol entsprechend der allgemeinen Formel

$$R^1\text{-}(O\text{—}A)_n\ OH$$

reagieren läßt, und daß man in einer zweiten Stufe das aus der ersten Stufe resultierende Produkt reagieren läßt mit einem monoprimären Amin entsprechend der allgemeinen Formel $R^2\text{-}(XB)_m NH_2$, wo $R^1$, A, n, $R^2$, X, B und m wie in Anspruch 1 definiert sind.

6. Verfahren gemäß Anspruch 5, dadurch gekennzeichnet, daß man in der zweiten Stufe ein monoprimäres Amin entsprechend der allgemeinen Formel $R^2\text{-}(X\text{—}B)_m NH_2$ in einem Verhältnis von 1,02 bis 1,2 Mole pro Mol des aus der ersten Stufe resultierenden Produktes verwendet.

7. Verfahren gemäß einem der Ansprüche 5 und 6, dadurch gekennzeichnet, daß man bei einer Temperatur von 65 bis 200°C arbeitet, wobei man das im Laufe der Reaktionen entstandene Wasser entfernt.

8. Verfahren gemäß einem der Ansprüche 5 bis 7, dadurch gekennzeichnet, daß man in einer einzigen Stufe arbeitet.

9. Treibstoff-Zusammensetzung, dadurch gekennzeichnet, daß sie einen Hauptanteil an Treibstoff umfaßt, und einen Anteil von 10 bis 500 ppm in Gewichtsanteilen eines N-substituierten Succinimids gemäß einem der Ansprüche 1 bis 4.

10. Treibstoff-Zusammensetzung gemäß Anspruch 9, dadurch gekennzeichnet, daß der Anteil an N-substituiertem Succinimid 20 bis 200 ppm in Gewichtsanteilen beträgt.